# EUROPÄISCHE PATENTSCHRIFT

(11) **EP 3 086 650 B1**
(45) Veröffentlichungstag und Bekanntmachung des Hinweises auf die Patenterteilung: **22.01.2020**
(21) Anmeldenummer: 14830961.0
(22) Anmeldetag: 23.12.2014
(51) Int. Cl.: A21D 8/04

(54) **VERFAHREN ZUR HERSTELLUNG EINES VORTEIGES ZUM BACKEN VON WEIZENBACKPRODUKTEN UNTER VERWENDUNG VON RÜCKBROT AUF WEIZENBASIS**
PROCESS FOR THE PRODUCTION OF A PRE-DOUGH FOR MAKING WHEAT-BASED BAKERY GOODS, WHICH USES STALED WHEAT BREAD
PROCÉDÉ POUR LA PRÉPARATION D'UNE PRÉ-PÂTE POUR DES PRODUITS DE BOULANGERIE À BASE DU BLÉ COMPRENANT L'UTILISAGE DES RESTES DU PAIN

(30) Priorität: 23.12.2013 EP 13199401
(43) Veröffentlichungstag der Anmeldung: 02.11.2016
(73) Patentinhaber: DIOSNA Dierks & Söhne GmbH, 49086 Osnabrück (DE)
(72) Erfinder: OLMS, Fridjof, 30916 Isernhagen (DE); LAUDE, Katrin, 30938 Burgwedel (DE); ZENSE, Torsten, 31311 Uetze (DE)
(74) Vertreter: dompatent von Kreisler Selting Werner - Partnerschaft von Patent- und Rechtsanwälten mbB
(86) Internationale Anmeldenummer: PCT/EP2014/079149
(87) Internationale Veröffentlichungsnummer: WO 2015/097216

(56) Entgegenhaltungen:
- SU-A1- 1 009 380
- "IsernHäger Brotfermentation", , 24. November 2009 (2009-11-24), XP054975349, Gefunden im Internet: URL:http://www.youtube.com/watch?v=jThVxku 8ajs [gefunden am 2014-03-11]
- "Lehrinfo zu den Isernhäger Verfahren", , 26. Juli 2013 (2013-07-26), Seiten 1-4, XP055106864, Gefunden im Internet: URL:http://isernhaeger.de/picts/1129905365 /Brotfermentation_und_Farster_Fuehrung.pdf [gefunden am 2014-03-11]
- ISERNHAEGER: "Einfluss der Zugabe von Brot auf die Sauerteiggärung // Influence of bread addition on sour dough fermentation", GETREIDE MEHL UND BROT, BOCHUM, DE, 1. Januar 1972 (1972-01-01), Seiten 207-208, XP009176815, ISSN: 0367-4177
- ISERNHAEGER LANDKOST MENGE: "Moderne Sauerteigführung über mehrere Stufen /// Modern sour dough method with several steps", BROT UND BACKWAREN, HAMBURG, DE, 1. Januar 1996 (1996-01-01), Seiten 36-38, XP009176814, ISSN: 0172-8180
- Lutz Geißler - Http://www.Ploetzblog.De/haftungsausschlus s/: "Freiberger Körnchen - Plötzblog - Selbst gutes Brot backen", , 6. November 2009 (2009-11-06), Seiten 1-2, XP055106877, Gefunden im Internet: URL:http://www.ploetzblog.de/2009/11/06/ge backen-freiberger-koernchen/ [gefunden am 2014-03-11]
- VOGELMANN S A ET AL: "Adaptability of lactic acid bacteria and yeasts to sourdoughs prepared from cereals, pseudocereals and cassava and use of competitive strains as starters", INTERNATIONAL JOURNAL OF FOOD MICROBIOLOGY, ELSEVIER BV, NL, Bd. 130, Nr. 3, 15. April 2009 (2009-04-15), Seiten 205-212, XP025980162, ISSN: 0168-1605, DOI: 10.1016/J.IJFOODMICRO.2009.01.020 [gefunden am 2009-01-29]
- F. MINERVINI ET AL: "Lactic Acid Bacterium and Yeast Microbiotas of 19 Sourdoughs Used for Traditional/Typical Italian Breads: Interactions between Ingredients and Microbial Species Diversity", APPLIED AND ENVIRONMENTAL MICROBIOLOGY, Bd. 78, Nr. 4, 15. Februar 2012 (2012-02-15), Seiten 1251-1264, XP055178050, ISSN: 0099-2240, DOI: 10.1128/AEM.07721-11
- ALICE V MORONI ET AL: "Biodiversity of lactic acid bacteria and yeasts in spontaneously-fermented buckwheat and teff sourdoughs", FOOD MICROBIOLOGY, ACADEMIC PRESS LTD, LONDON, GB, Bd. 28, Nr. 3, 20. Oktober 2010 (2010-10-20), Seiten 497-502, XP028366427, ISSN: 0740-0020, DOI: 10.1016/J.FM.2010.10.016 [gefunden am 2010-10-28]
- Andreas Ranft: "Die richtige Anwendung- Backversuch Restbrot", Artisan, 1. April 2009 (2009-04-01), Seiten 10-15, XP055107129, Gefunden im Internet: URL:http://isernhaeger.de/picts/90380f9898 f742c37ef0c0794bc95ef8/04_2009_Artisan_Res tbrot.pdf [gefunden am 2014-03-12]

## Beschreibung

Gegenstand der vorliegenden Erfindung ist eine Starterkultur zur Herstellung von Sauerteig, Lactobacillen, ein Verfahren zur Herstellung eines auf Weizen basierenden Sauerteiges unter Zugabe von Rückbrot, und ein Vorteig zur Herstellung von Weizenbackprodukten erhältlich nach dem erfindungsgemäßen Verfahren.

Rückbrot ist ein hochwertiges Produkt mit wertvollen Inhaltsstoffen. In Wikipedia wird berichtet, dass Rückbrot als Tierfutter verwendet oder durch alkoholische Gärung zu Biotreibstoff verarbeitet werden kann. Geringe Mengen gelangen in karitative Einrichtungen, wie Tafel (Organisation), oder in die private Tiermast. Im Bäckereihandwerk werden Weißbrot und Brötchen, welche keine Saaten, Gewürze oder Trockenfrüchte enthalten, zum Teil trocken und warm gelagert und zu Paniermehl verarbeitet. Bei der Herstellung von Vollkornbroten werden zum Teil Brotabschnitte und Rückbrot zu Brühstück oder Quellstück weiterverarbeitet, welche die Funktion von Quellmehl erfüllen. Das Produkt wird saftiger und die Struktur der Krume bei Produkten aus ganzen Körnern verbessert. In der Industrie und Handwerk ist die Zugabe von Rückbrot bei Mischbroten durchaus üblich, um die Qualität des Produktes und die Kostensituation zu verbessern. Die Verarbeitung zu Futtermehl ist verbreitet. In den Niederlanden ist dazu eine Systemlogistik entwickelt worden. Seit Ende 2006 ist in Deutschland die Verfütterung von Küchen- und Speiseresten an Nutztiere verboten (EU-Richtlinie 1774/2002). Dieses Verbot gilt nicht für Rückbrot und Teigwaren. Allerdings sind die für Futtermittel geltenden gesetzlichen Regelungen anzuwenden. Die TU-Berlin hat mit verschiedenen Unternehmen ein Verfahren entwickelt, mit dem aus Rückbrot Hefe produziert wird. Altbrot wird auch als Brennstoff verwendet. Die Verwendung von verkehrsfähigem hygienisch einwandfreiem Brot bei der Brotherstellung ist in Deutschland gemäß den Leitsätzen für Brot und Kleingebäck üblich [Referenz 1] (in anderen europäischen Ländern können abweichende Regelungen bestehen) Bei Brot mit überwiegendem Weizenanteil sind bis zu 6 Prozent Verwendung von Restbrot oder Rückbrot, bezogen auf die Gesamtmenge des verwendeten Getreides und/oder der Getreideerzeugnisse, erlaubt, bei überwiegendem Roggenanteil bis zu 20 Prozent. Die Zugabe von Wasser und anderen Rezepturbestandteilen zur Herstellung von Backprodukten berücksichtigend, können damit 3 bis 3,5 Gewichts-Prozent bei Weizen(misch)broten und 10 bis 11 Gewichts-Prozent bei Roggen(misch)broten als Rückbrot wiederverwertet werden. Forderung der Leitsätze [1] ist, dass das "mitverwendete Brot ... im Enderzeugnis mit bloßem Auge nicht erkennbar (ist)".

Bekannt sind Verfahren, bei denen eine Zugabe von Rückbrot über getrocknetes Misch-, Roggenmisch- und Roggenbrot erfolgt. Eine bekannte Vorgehensweise verwendet 10kg getrocknetes, vermahlenes Brot und weicht dieses mit der 1,2-fachen bis 5-fachen Menge an Wasser ein. Im Verlauf einer Stehzeit über Nacht quillt die Mischung auf.

Ein Nachteil der Verfahren, in denen lediglich eine Verquellung erfolgt, besteht darin, dass in Broten, die mit solchen Aufschlämmungen gebacken werden, zu einem gewissen Anteil bis zu linsengroße Partikel der ursprünglichen Brote sichtbar in der Krume wiedergefunden werden können und teilweise als vermeintlicher Fremdkörper, zumindest aber als Unreinheit empfunden werden können. Die Deutsche Landwirtschafts-Gesellschaft DLG bewertet solche Partikel in den nationalen Qualitätsbeurteilungen von Brot als "Flecken" und bewertet sie als Brotfehler.

Da Brot einen guten Nährboden nicht nur für Nutzorganismen wie Milchsäurebakterien und Hefen darstellt, sondern auch für potenzielle Verderbniskeime, ist insbesondere bei Raumtemperatur Restbrotaufschlämmung innerhalb weniger Stunden zu verarbeiten.

Generell gilt, dass jedem Verfahren, welches die gesetzlich erlaubten Anteile an Lebensmitteln, hier Brot, in den Herstellprozess zurückführt, zu eigen sein muss, dass Maßnahmen der Hygiene und des HACCP (Hazard Analysis and Critical Control Point, deutsch: "Gefahrenanalyse und kritische Kontrollpunkte"; beschreibt ein System zur Festlegung und dokumentierten Kontrolle von Kontrollpunkten im Prozess, die gemäß einer Risikobewertung die Sicherheit für den menschlichen Verzehr der im Prozess hergestellten/verarbeiteten/vertriebenen Produkte gewährleisten sollen) Voraussetzung sind, um überhaupt eine Rückführung von Material durchführen zu können.

Die Problematik der Verwertung von Brot wird von der Landesanstalt für Umwelt, Messungen und Naturschutz Baden-Württemberg betrachtet. [Ref. 2]. Es wird ausgeführt, dass "bei den großen Filialbäckern ausschließlich Produktionsreste wie beispielsweise Fehlchargen zurückgeführt" werden, weil die "für eine Weiterverarbeitung erforderlichen Hygienestandards ... von Retouren [Hinweis: aus Filialen] nicht erreicht (werden)".

In Linienproduktionen gilt der Anfall von 1 bis zu 3 Prozent Brot, welches aus Qualitätsmängeln heraus als nicht verkaufsfähig beurteilt und damit einer Rückbrotverwertung zugeführt wird, als realistische Größenordnung. Bei einem Ausstoß von 1000 Broten je Stunde entspricht das einem Verlust von 10 bis 30 Broten je Stunde.

Verluste bzw. Reste aus der Brotherstellung entstehen auch dann, wenn Brote bereits vorgeschnitten werden, bevor sie, zumeist verpackt, in den Verkauf gelangen. Typische "Abfall"mengen bei Schnittbrot entstehen beispielsweise durch die Endstücke, die oftmals nicht mitverpackt werden. Typisch sind auch "Fehl-Schnitte" bei leichten Formabweichungen des Naturprodukts Brot, bei dem Abbrechen harter Kruste etc.

Man erkennt auch an diesen Beispielen, warum die Auslotung der Möglichkeiten zur Verwertung von Rückbrot ein wichtiges Thema ist.

SU 1009380 A offenbart ein Verfahren zur Herstellung von Weißbrot. Vogelmann et al. in International Journal of Food Microbiology, 130 (2009) 205-212 untersuchen verschiedene Lactobazillen und Hefen als Starter.

F. Minervini et al. in Applied and Environmental Microbiology, 78 (2012) 1251-1264 untersuchen Lactobazillen und Hefen in Sauerteigen.

A. V. Moroni et al. in Food Microbiology 28 (2011) 497-502 untersuchen Lactobazillen und Hefen in Buchweizensauerteig.

Ein der Erfindung zu Grunde liegendes technisches Problem besteht in der Bereitstellung eines Verfahrens, mit dem Reste aus Weizenbackprodukten einem Backprozess wieder zugeführt werden können Ein weiteres technisches Problem ist die Vermeidung sichtbarer Flecken im Brot. Eine weitere Aufgabe der vorliegenden Erfindung besteht in der Bereitstellung von Mitteln zur Durchführung des Verfahrens, insbesondere die Schaffung von Starterkulturen zur Fermentierung und Herstellung eines Sauerteigs.

Gelöst werden die Probleme erfindungsgemäß durch ein Verfahren zur Herstellung eines fermentierten Vorteiges aus Rückbrot als Bestandteil von Teigen zum Backen von Weizenbackprodukten gemäß der Ansprüche.

Zur Durchführung des erfindungsgemäßen Verfahrens wird eine Starterkultur zur Herstellung von Sauerteig, enthaltend eine Mischung von Lactobacillus paracasei BSB 2 DSM 28104 und Lactobacillus gallinarum BSB 1 DSM 28103 verwendet.

In einer Ausführungsform der erfindungsgemäßen Starterkultur kann diese Lactobacillus paracasei BSB 2 DSM 28104 und Lactobacillus gallinarum BSB 1 DSM 28103 aufweisen sowie weitere Lactobacillen und Hefen, sowie Getreidemahlprodukte zur Erzeugung einer eingedickten, lagerfähigen Mischung, die verkaufsfähig ist.

In einer anderen Ausführungsform der erfindungsgemäßen Starterkultur kann die Starterkultur mindestens eine weitere der im Folgenden genannten Komponenten Lactobacillus brevis, Lactobacillus delbrueckii, Lactobacillus plantarum und Saccharomyces cerevisiae, sowie Roggenschrot, Roggenmehl und Wasser aufweisen.

In noch einer anderen Ausführungsform der erfindungsgemäßen Starterkultur kann diese enthalten 0,005 bis 0,2 Gewichtsprozent, insbesondere etwa 0,02 Gewichtsprozent der Mischung von Mikroorganismenkulturen, darin 0,005 bis 0,2 Gewichtsprozent, insbesondere etwa 0,018 Gewichtsprozent von Lactobacillus paracasei BSB 2 DSM 28104 und Lactobacillus gallinarum BSB 1 DSM 28103, sowie 40 bis 60 Gewichtsprozent Roggenschrot, insbesondere 45 bis 50 Gewichtsprozent Roggenschrot, 20 bis 30 Gewichtsprozent Roggenmehl, insbesondere 25 Gewichtsprozent Roggenmehl und 20 bis 30 Gewichtsprozent, insbesondere 25 Gewichtsprozent Wasser.

Gegenstand der vorliegenden Erfindung sind auch Lactobacillus paracasei BSB 2 Eingangsnummer DSM 28104 sowie Lactobacillus gallinarum BSB 1 Eingangsnummer DSM 28103. Diese Stämme wurden am 19.12.2014 bei der Sammlung Leibniz Institut DSMZ-Deutsche Sammlung von Mikroorganismen und Zellkulturen GmbH, Inhoffenstraße 7B, D-38124 Braunschweig, Deutschland, unter den Bedingungen des Budapester Vertrages hinterlegt.

Das erfindungsgemäße Verfahren weist die folgenden Schritte auf:
a) In einer ersten Stufe Ansetzen eines Sauerteigs durch Mischen von ungefähr 100 Gewichtsteilen Weizenmehl mit 100-200 Gewichtsteilen Wasser und Animpfen mit einer mit Getreidemahlprodukten eingedickten erfindungsgemäßen Starterkultur
   in einem Temperaturbereich von 20-35°C, bevorzugt bei 26-32 °C, die erhaltene erste Mischung für eine Zeitdauer von 12-36 h, bevorzugt 20-28 h reifen lassen.
b) In einer zweiten Stufe Herstellung einer Mischung aus Weizenmehl, einem Weizenbrotkontingent und Wasser, umfassend eine Menge von 75-135 Gewichtsteilen, bevorzugt 75-120 Gewichtsteilen, Weizenmehl; davon zu 15-75 Gewichtsteilen, bevorzugt 30-75 Gewichtsteilen Weizenbrotkontingent, die insbesondere für eine Zeitdauer von 10-120 min, bevorzugt 30-60 min, unter Rühren in zwei Schritten mit 150 Gewichtsteilen Wasser angemischt wird, wobei das Produkt eine Temperatur in einen Temperaturbereich von 20-35°C aufweisen sollte.
c) Vermischen eines Teils des in der ersten Stufe gemäß Schritt a) gewonnen Sauerteigs mit einem größeren Teil der zweiten Mischung zur Initialisierung der Fermentation der zweiten Stufe.
d) Reifenlassen der in Schritt c) erhaltenen kombinierten Mischung für eine Zeitdauer von 24-72 h, bevorzugt 36-60h, unter Erhalt eines Vorteiges als Bestandteil von Teigen zur Herstellung der Weizenbackprodukte.

Die Verhältnisse der Zugabemengen aus a) und b) für die kombinierte Mischung in Schritt c) können vom Fachmann in einfacher Weise, unter Anwendung seines Fachwissens bestimmt und eingestellt werden. Typischerweise werden sie in Abhängigkeit sowohl von der Beschaffenheit der ersten Stufe aus a) als auch der Beschaffenheit der zweiten Stufe aus b) gewählt. Die Wahl der in die Mischung aus a) und b) einzubringenden Menge von a) hängt beispielsweise von Wachstumsvorteilen für einige Mikroorganismen der Mischkultur gegenüber anderen bei Nutzung der Spannbreite der Temperatur ab, wobei die Eigenschaft, die Fermentation der zweiten Stufe zu initialisieren, insbesondere mit deren erfindungsgemäßer Besonderheit der Verwertung und des Aufschlusses von Rückbrot, gewahrt bleibt. Im Falle der zweiten Stufe hängt die Wahl zum Beispiel von der Zusammensetzung des eingesetzten Weizenbrotkontingents als Rückbrot ab. Die Zusammensetzung des Weizenbrotkontingents als Rückbrot gibt der Anwender des Verfahrens aufgrund seines Artikel-Mixes vor, so dass daraufhin das Zugabeverhältnis gemeinsam definiert wird.

Typischerweise werden im erfindungsgemäßen Verfahren zur Herstellung der zweiten Stufe 5,0 bis 20,0 Gewichtsteile der ersten Stufe a) mit 100 Gewichtsteilen der zweiten Mischung b) gemischt.

Dem Problem des potenziellen Nährbodens auch für Verderbniskeime wird dadurch begegnet, dass eine gezielte Vorteigfermentation den pH-Wert so stark absenkt, dass sehr lange natürliche Lagerstabilität erreicht wird.

Diese Stabilität wird durch die Kombination der vom Erfinder selbst entwickelten Starterkultur in Verbindung mit den dafür entwickelten Führungsparametern erreicht.

Generell gilt, dass eine Fermentation durch das stetige Absinken des pH-Werts Verderbnisorganismen in ihrer Entwicklung hemmt und schon dadurch die möglichen, überhaupt erreichbaren Keimzahlen dieser Organismen im Ansatz klein gehalten werden.

Das erfindungsgemäße Verfahren ermöglicht es auf natürliche Weise besonders niedrige pH-Werte, bis zu pH 3,4, zu erreichen, wodurch eine natürliche Lagerstabilität des Vorteiges bis zu 96 Stunden, maximal bis zu 144 Stunden, selbst ohne Kühlung, erreicht wird.

Ein wesentlicher Vorteil des erfindungsgemäßen Verfahrens besteht darin, dass die Verwendung von Rückbrot nicht allein durch mechanische Verfahren, gestützt durch Mechanismen der Verquellung, erfolgt, sondern eine Fermentation initiiert wird, die durch Auswahl der Mikroorganismen sowie entsprechender Parametervorgaben gesteuert und zielgerichtet abläuft.

Charakteristisch ist dabei, dass die Fermentation Abbau- und Umwandlungsprozesse beinhaltet. Erstere führen unter anderem zum verstärkten Aufschluss der Bestandteile des Rückbrots, letztere zur Bildung natürlicher Milch- und Essigsäure, welche nach Abschluss des Reifeprozesses sowohl die primär geschmacksbildenden als auch, durch pH-Wert-Absenkung, die verlängerte Lagerfähigkeit des Sauerteiges ermöglichen. Die natürliche Fermentation ist zugleich maßgebend für die Anwendung des Begriffs Natursauerteig, der auch für das erfindungsgemäße Verfahren adaptierbar ist.

In einer Ausführungsform des erfindungsgemäßen Verfahrens wird in den Sauerteigstufen Weizenmehl vom Typ 550 nach DIN 10355 eingesetzt, was einem Aschegehalt von 510 bis 630 mg/100 g Mehl entspricht. In einer Abwandlung beträgt der Ausmahlungsgrad des Mehles 640 bis 900 mg/100 g, in einer weiteren Ausführungsform 910 bis 1200 mg/100 g, in besonderen Ausführungsformen 1210 bis 2100 mg/100 g Mehl.

In einer weiteren Ausführungsform reift erfindungsgemäß die erste Mischung für eine Zeitdauer von 12-36 h, bevorzugt 20-28 h, Temperaturbereich von 20-35°C, bevorzugt bei 26-32 °C.

Im erfindungsgemäßen Verfahren werden insbesondere in der ersten Stufe 100 Gewichtsteile Weizenmehl mit 100-200 Gewichtsteilen Wasser gemischt.

Typischerweise werden im erfindungsgemäßen Verfahren zur Herstellung der zweiten Stufe 5,0 bis 20,0 Gewichtsteile der ersten Stufe mit 100 Gewichtsteilen der zweiten Mischung gemischt werden, wobei diese bei einer Zeitdauer von 24-72 h, bevorzugt 36-60 h bei 20-35°C, bevorzugt bei 26-32 °C reifen gelassen wird.

Der mit dem erfindungsgemäßen Verfahren herstellbare Vorteig als Bestandteil des Hauptteiges kann vorteilhafterweise zum Backen von Weizenbackprodukten eingesetzt werden.

Gegenstand der Erfindung ist auch ein mittels des erfindungsgemäßen Verfahrens erhältlicher befristet haltbarer Vorteig für die Direktzugabe zu Teig zum Backen von Weizenbackprodukten.

Die Erfindung wird im folgenden Ausführungsbeispiel näher erläutert.

### Brotfermentation Weizen

1. Stufe (in einem isolierten Behältnis, z.B. einem IsoFermenter der Fa. IsernHäger) :
   - 15 Liter temperiertes Wasser + 10 Kg Weizenmehl Type 550 vermischen; oder jede andere Mischung von Wasser und Mehl, die diesem Verhältnis entspricht,
   - Soll-Temperatur nach dem Vermischen: 29 °C
   - Die Mischkultur, die handelsüblich mit Backschrot und Mehl eingedickt und lagerfähig gemacht worden ist, und im Folgenden als Starter Brotfermentation Weizen bezeichnet wird, aus der Kühlung nehmen und in einer Schale zerkrümeln.
   - Starter Brotfermentation Weizen unter das temperierte Wasser / Mehl Gemisch geben
   - Reifezeit: 24 Stunden
2. Stufe (in einer Anlage B300 der Fa. IsernHäger):
   - Als erstes gut 80% des benötigten temperierten Wassers zugeben, anschließend 50% der Rückbrotmenge in die Anlage geben und ein Zerkleinerungs- und Mischprogramm starten.
   - Nach 5 Minuten die restliche Brotmenge in die Anlage geben und Anlage erneut starten.
   - Nach weiteren 5 Minuten die erforderliche Weizenmehlmenge zugeben und Anlage erneut starten.
   - 10 min rühren lassen.
   - Anschließend die vorbereitete 1. Stufe dazugeben sowie die restlichen knapp 20% Wasser. Temperatur nach dem Vermischen aller Zutaten sollte bei 30°C liegen.
   - Reifezeit: 48 Stunden

Der hergestellte fermentierte Vorteig ist ein Sauerteig, der als Bestandteil eines Hauptteiges vorteilhafterweise zum Backen von Weizenbackprodukten eingesetzt werden kann, aber auch in Weizen-Roggen-Misch-Backprodukten. Erfindungsgemäß werden entsprechend der Prozessbeschreibung durch die Kombination der vom Erfinder selbst entwickelten Starterkultur in Verbindung mit den eigens entwickelten Führungsparametern auf natürliche Weise besonders niedrige pH-Werte, bis zu pH 3,5, erreicht, wodurch eine natürliche Lagerstabilität des Vorteiges bis zu 96 Stunden, maximal bis zu 144 Stunden, selbst ohne Kühlung, erreicht wird.

Gegenstand der Erfindung ist daher auch ein mittels des erfindungsgemäßen Verfahrens erhältlicher bis zu 144 Stunden haltbarer Vorteig für die Direktzugabe zu Teig zum Backen von Weizenbackprodukten.

### Beispiel 1:

Die Anwendung des zweistufigen Verfahrens mit konkreten Mengenangaben und Fermentationsparametern kann beispielhaft wie folgt ausgeführt werden:
15,000 kg Wasser und 10,000 kg Weizenmehl, mit einem Aschegehalt von 510 bis 630 mg/100 g Mehl, werden vermischt. Die Wassertemperatur wird dabei so gewählt, dass die Temperatur des Gemisches 29 °C +1,5/-0,5 °C beträgt. 0,750 kg des Starters Brotfermentation Weizen werden aus der Kühlung genommen und in einer Schale zerkrümelt. Anschließend wird der Starter unter das Wasser / Mehl Gemisch gerührt. Dies sollte in einem möglichst gut temperaturisolierten, verschließbaren Behälter erfolgen, wie ihn für diesen Maßstab zum Beispiel ein Isofermenter der Fa. IsernHäger bietet, dessen Fassungsvolumen 70 Liter beträgt.

Die auf diese Weise bereitete 1. Stufe erfährt nun eine Fermentationszeit von 24 Stunden. Die Fermentationstemperatur wird sich bei korrekter Ansatztemperatur und gut isoliertem Behältnis typischerweise zwischen 29 und 33 °C bewegen.

Die nach diesem Verfahren angesetzte 1. Stufe ist bis zu 48 Stunden für den Ansatz einer 2. Stufe verwendbar.

Für den Ansatz der 2. Stufe wird in diesem Beispiel eine Fermentationsanlage des Typs B300 der Fa. IsernHäger eingesetzt. Es handelt sich dabei um einen 300 kg fassenden Edelstahlbehälter mit einem Fassungsvolumen von ungefähr 390 Litern, der ein Messerrührwerk mit Stator und Rotor enthält, das in der Lage ist, Rückbrot-Laibe als auch Brotscheiben zu zerkleinern, dementsprechend mit einem leistungsfähigen Motor ausgestattet ist, sowie die Rührwerksansteuerung und den Prozessverlauf nach verschiedenen Parametern ermöglicht.

In den Fermenter werden zunächst 120 kg Wasser dosiert, derart temperiert, dass unter Berücksichtigung der Temperatur aller Zutaten letztendlich eine Mischtemperatur von 30 °C +/-2 °C erreicht wird. Anschließend wird die Hälfte des zu verarbeitenden Rückbrots auf Weizenbasis, in diesem Beispiel 37,5 kg, in den Fermenter gegeben. In einem Zerkleinerungs- und Mischprogramm wird das Rückbrot über die Zeitdauer von 5 Minuten grob zerkleinert. Nun wird die zweite Hälfte, entsprechend 37,5 kg, Rückbrot hineingegeben, und das Zerkleinerungs- und Mischprogramm ist erneut für eine Laufzeit von 5 Minuten in Betrieb.

Jetzt erfolgt die Zugabe von 75 kg Weizenmehl, und die Anlage führt eine zehnminütige Mischphase aus.

Als letzter Schritt erfolgen die Beimengung des Anteils der 1. Stufe, in diesem Beispiel 25,75 kg, und schließlich die Restmenge von 30 kg Wasser. Zieltemperatur der kombinierten Mischung sind, wie oben angegeben, 30 °C +/-2 °C.

Die auf diese Weise bereitete 2. Stufe durchläuft in diesem Anwendungsbeispiel nun eine Fermentationszeit von 60 bis 66 Stunden. Je nach Raumtemperatur wird die Fermentationstemperatur typischerweise zwischen 25 und 33 °C liegen.

Die für die Verwendung des Vorteiges in Rezepturen zur Herstellung von Weizenbackprodukten wichtige fermentative Reife drückt sich in der Versäuerung aus. Die Versäuerung gilt als abgeschlossen, wenn der pH-Wert und die Menge gebildeter Säure, ermittelt als Säuregrad, in einer grafischen Auftragung über der Zeitachse begonnen haben, ein Plateau zu bilden. Von nun an ermöglicht die Lagerstabilität, die erfindungsgemäß unter Verwertung des Rückbrotanteils erreicht wird, die lange Verarbeitungsfähigkeit des Vorteiges bei gleichen Verarbeitungseigenschaften.

Selbst eine kleine Nachreifung über diesen Zeitpunkt hinaus, begründet durch die aktive Kultur, ändert die Teigqualitäten und späteren Gebäckqualitäten nur noch so geringfügig, dass Sie aus Sicht des Konsumenten sensorisch nicht erfassbar sind.

Typischerweise, abhängig von der beschriebenen Entwicklung der Mischkultur sowie der Zusammensetzung der als Rückbrot eingesetzten Weizenbackprodukte, sind das Plateau bzw. der Beginn der Verarbeitungszeit dadurch gekennzeichnet, dass der erreichte pH-Wert kleiner als 4,0 ist, und der erreichte Säuregrad größer als 16 ist. Die natürliche Lagerstabilität des derart gesäuerten Vorteigs der 2. Stufe beträgt bis zu 144 Stunden.

Eine Teigrezeptur zur Herstellung eines Weizenbackproduktes unter Verwendung des nach dem beschriebenen Verfahren erzeugten Vorteigs zeigt nachfolgende Tabelle. Im vorliegenden Beispiel ist eine moderate Zugabemenge von 6 Teilen Weizenmehl plus 6 Teilen Wasser, bezeichnet als Vorteig "Brotfermentation Weizen", bezogen auf 100 Teile des eingesetzten Weizenmehls gewählt worden. Der Salzgehalt wurde nach aktuellen Empfehlungen auf 1,5 Prozent bezogen auf Mehl gesetzt. Die moderate Zugabemenge des Vorteigs in diesem Beispiel ist dann geeignet, wenn die Erwartungshaltung des Endverbrauchers in Bezug auf den Geschmack des Weizenbrotes wie zum Beispiel bei einem Sandwichbrot eher von einem milden Brotgeschmack ausgeht.

### Weizenbrot

### Basis:

### 100 kg Gesamtmehl

| **Teigzutaten** | **in kg** | **Herstellungs-Parameter** | |
|---|---|---|---|
| **Teig** | | Teigruhezeit: | 48 Min. |
| Vorteig "Brotfermentation Weizen" | | Anteil Vorteig, ausgedrückt als | |
| (bei einem Mehl-Wasser-Verhältnis von 1:1) | 12,000 | Anteil Weizenmehl, fermentiert: | 6,0 % |
| | | Teigausbeute: | 150,5 |
| Weizenmehl | 94,000 | Teigtemperatur: | 27-28 |
| Hefe | 3,400 | Hefezugabe in %: | 3,4 % |
| Salz | 1,500 | Salzgehalt in %: | 1,5 % |
| Wasser | 44,500 | Teigeinlage für 1 Brot: | 0,900 kg |
| | | Resultierende Anzahl Brote (Gewicht nach Ausbackverlust | |
| **Teiggewicht:** | **155,400** | 0,750 kg): | 172,7 |
| - | | - | |

| **Aufarbeitung:** | **Knetzeiten (Spiralkneter):** | |
|---|---|---|
| rund- und langwirken | langsam: | 3 min |
| | schnell: | 10 min |
| | | |

| | **Stückgare:** | |
|---|---|---|
| | Temperatur: | 35 °C |
| | Luftfeuchte: | 85 % |
| | Zeit: | 65 min |

| | **Backprozess:** | |
|---|---|---|
| | Backtemperatur Anfang: | 250°C |
| | Backtemperatur Ende: | 210°C |
| | Zeit: | 33 min |

Entsprechend dieser Rezeptur wurden in den 3 Teilen (von insgesamt 6 Teilen) Vorteig, die aus dem Rückbrot kommen, rechnerisch 4 Brote zu je 0,750 kg verarbeitet, was bezogen auf 172,7 Brote insgesamt die Verwertung eines Rückbrotanteils von 2,3 Prozent bedeutet.

Der Anwender des Verfahrens kann den Einsatz eines Vorteigs aus dem Verfahren jedoch produktbezogen ausdehnen. In einem herzhaften Weizengebäck wie Ciabatta könnte der Anteil beispielsweise auf bis zu 12 von 100 Teilen Weizenmehl ausgedehnt werden, respektive auf bis zu 6 Teile Rückbrot. Letztendlich ist die Zugabemenge dem Anwender überlassen, sofern er die Richtlinien der Verwertung von Rückbrot (siehe Einleitung Seite 1, Zeile 28ff) beachtet.

### Referenzen:

[1] Bundesministerium für Ernährung, Landwirtschaft und Verbraucherschutz (Hrsg.): Leitsätze des Deutschen Lebensmittelbuchs für Brot und Kleingebäck vom 19.10.1993, zuletzt geändert am 19.09.2005
[2] Landesanstalt für Umwelt, Messungen und Naturschutz Baden-Württemberg: Ideen für mögliche Abfallvermeidung in Baden-Württemberg. Kostenloser Download unter: www.lubw.badenwuerttemberg.de, ISBN 978-3-88251-374-5, Stand Mai 2013

## Patentansprüche

1. Starterkultur zur Herstellung von Sauerteig, enthaltend eine Mischung von *Lactobacillus paracasei* BSB 2 DSM 28104 und *Lactobacillus gallinarum* BSB 1 DSM 28103.

2. Starterkultur nach Anspruch 1 enthaltend *Lactobacillus paracasei* BSB 2 DSM 28104 und *Lactobacillus gallinarum* BSB 1 DSM 28103 sowie weitere Lactobacillen und Hefen, die gemeinsam eine Mischung von Mikroorganismenkulturen bilden sowie Getreidemahlprodukte.

3. Starterkultur nach Anspruch 1 oder 2, enthaltend als Mischung von Mikroorganismenkulturen *Lactobacillus paracasei* BSB 2 DSM 28104 und *Lactobacillus gallinarum* BSB 1 DSM 28103, *Lactobacillus brevis, Lactobacillus delbrueckii, Lactobacillus plantarum, Saccharomyces cerevisiae,* sowie Roggenschrot, Roggenmehl und Wasser.

4. Starterkultur nach mindestens einem der Ansprüche 1 bis 3, enthaltend 0,005 bis 0,2 Gewichtsprozent, insbesondere etwa 0,02 Gewichtsprozent der Mischung von Mikroorganismenkulturen, darin 0,005 bis 0,2 Gewichtsprozent, insbesondere etwa 0,018 Gewichtsprozent von *Lactobacillus paracasei* BSB 2 DSM 28104 und *Lactobacillus gallinarum* BSB 1 DSM 28103, sowie 40 bis 60 Gewichtsprozent Roggenschrot, insbesondere 45 bis 50 Gewichtsprozent Roggenschrot, 20 bis 30 Gewichtsprozent Roggenmehl, insbesondere 25 Gewichtsprozent Roggenmehl und 20 bis 30 Gewichtsprozent, insbesondere 25 Gewichtsprozent Wasser.

5. *Lactobacillus paracasei* BSB 2 DSM 28104.

6. *Lactobacillus gallinarum* BSB 1 DSM 28103.

7. Verfahren zur Herstellung eines Vorteiges als Bestandteil von Teigen zum Backen von Weizenbackprodukten unter Verwendung eines Weizenbrotkontingents mit den folgenden Schritten:
a) In einer ersten Stufe Ansetzen eines Sauerteigs durch Mischen von ungefähr 100 Gewichtsteilen Weizenmehl mit 100-200 Gewichtsteilen Wasser und Animpfen mit einer mit Getreidemahlprodukten eingedickten Starterkultur nach mindestens einem der Ansprüche 1 bis 4, deren Temperaturbereich bei 20-35°C liegt, und die erhaltene erste Mischung für eine Zeitdauer von 12-36 h reift,
b) In einer zweiten Stufe Herstellung einer Mischung aus Weizenmehl, einem Weizenbrotkontingent und Wasser, umfassend eine Menge von 75-135 Gewichtsteilen; davon zu 15-75 Gewichtsteilen Weizenbrotkontingent, die insbesondere für eine Zeitdauer von 10-120 min, unter Rühren in zwei Schritten mit 150 Gewichtsteilen Wasser angemischt wird, wobei das Produkt eine Temperatur in einem Temperaturbereich von 20-35°C aufweist,
c) Vermischen eines Teils des in der ersten Stufe gemäß Schritt a) gewonnen Sauerteigs mit einem größeren Teil der zweiten Mischung zur Initialisierung der Fermentation der zweiten Stufe,
d) Reifenlassen der in Schritt c) erhaltenen kombinierten Mischung für eine Zeitdauer von 24-60 h, unter Erhalt eines Vorteiges als Bestandteil von Teigen zur Herstellung der Weizenbackprodukte.

8. Verfahren nach Anspruch 7, wobei das Weizenmehl ein Mehl vom Typ 550 nach DIN 10355 ist, was einem Aschegehalt von 510 bis 630 mg/100 g Mehl entspricht, Mehl mit einem Ausmahlungsgrad von 640 bis 900 mg/100 g.

9. Verfahren nach mindestens einem der Ansprüche 7 oder 8, wobei die kombinierte, zweite Stufe bei einer Zeitdauer von 20-48 h, bei 20-35°C reifen gelassen wird.

10. Verfahren nach mindestens einem der Ansprüche 7 bis 9, wobei zur Herstellung der kombinierten, zweiten Stufe 2,5 bis 15,0 Gewichtsteile der ersten Stufe mit 100 Gewichtsteilen der zweiten Mischung gemischt werden.

11. Verfahren nach einem der Ansprüche 7 bis 10, bei dem in einem rein auf Fermentation beruhenden Prozess sich ein pH-Wert von pH 3,5 einstellt, und eine Lagerstabilität von maximal bis 144 Stunden ohne Kühlung erreicht wird.

12. Vorteig als Bestandteil von Teigen zur Herstellung von Weizenbackprodukten erhältlich nach einem Verfahren gemäß Anspruch 7.

## Claims

1. A starter culture for producing sourdough, comprising a mixture of *Lactobacillus paracasei* BSB 2 DSM 28104 and *Lactobacillus gallinarum* BSB 1 DSM 28103.

2. The starter culture according to claim 1, comprising *Lactobacillus paracasei* BSB 2 DSM 28104 and *Lactobacillus gallinarum* BSB 1 DSM 28103 as well as other lactobacilli and yeasts, which commonly form a mixture of microorganism cultures, and ground cereal products.

3. The starter culture according to claim 1 or 2, comprising, as a mixture of microorganism cultures, *Lactobacillus paracasei* BSB 2 DSM 28104 and *Lactobacillus gallinarum* BSB 1 DSM 28103, *Lactobacillus brevis, Lactobacillus delbrueckii, Lactobacillus plantarum, Saccharomyces cerevisiae,* as well as rye meal, rye flour and water.

4. The starter culture according to at least one of claims 1 to 3, comprising from 0.005 to 0.2% by weight, especially about 0.02% by weight, of said mixture of microorganism cultures, including from 0.005 to 0.2% by weight, especially about 0.018% by weight, of *Lactobacillus paracasei* BSB 2 DSM 28104 and *Lactobacillus gallinarum* BSB 1 DSM 28103, and from 40 to 60% by weight of rye meal, especially from 45 to 50% by weight of rye meal, from 20 to 30% by weight of rye flour, especially 25% by weight of rye flour, and from 20 to 30% by weight, especially 25% by weight, of water.

5. *Lactobacillus paracasei* BSB 2 DSM 28104.

6. *Lactobacillus gallinarum* BSB 1 DSM 28103.

7. A process for producing a starter dough as a component of doughs for baking baked wheat products using a wheat bread contingent, comprising the following steps:
a) in a first step, preparing a sourdough by mixing 100 weight parts of wheat flour with 100-200 weight parts of water, and inoculating with a starter according to any of claims 1 to 4, thickened with ground cereal products, whose temperature range is from 20 to 35 °C, and allowing the first mixture obtained to ripen for a period of 12-36 h;
b) in a second step, preparing a mixture of wheat flour, a contingent of wheat bread and water, comprising an amount of 75-135 weight parts, of which 15-75 weight parts is a contingent of wheat bread, which is mixed with 150 weight parts of water, in particular, for a period of 10-120 min with stirring in two steps, wherein the product has a temperature within a temperature range of 20-35 °C;
c) mixing part of the sourdough obtained in the first step according to step a) with a greater part of the second mixture to initiate the fermentation of the second step;
d) allowing the combined mixture obtained in step c) to ripen for a period of 24-60 h, to obtain a starter dough as a component of doughs for producing said baked wheat products.

8. The process according to claim 7, wherein said wheat flour is a flour of type 550 according to DIN 10355, which corresponds to an ash content of from 510 to 630 mg/100 g of flour, flour with an extraction rate of from 640 to 900 mg/100 g.

9. The process according to at least one of claims 7 or 8, wherein the combined second step is allowed to ripen at 20-35 °C for a period of 20-48 h.

10. The process according to at least one of claims 7 to 9, wherein from 2.5 to 15.0 weight parts of the first step is mixed with 100 weight parts of the second mixture to prepare the combined second step.

11. The process according to any of claims 7 to 10, in which a pH value of pH 3.5 is obtained and a storage stability of maximally up to 144 hours is achieved without cooling in a process based on fermentation only.

12. A starter dough as a component of doughs for preparing baked wheat products, obtainable by a process according to claim 7.

## Revendications

1. Culture d'amorce pour la production de levain, comprenant un mélange de *Lactobacillus paracasei* BSB 2 DSM 28104 et *Lactobacillus gallinarum* BSB 1 DSM 28103.

2. Culture d'amorce selon la revendication 1 comprenant *Lactobacillus paracasei* BSB 2 DSM 28104 et *Lactobacillus gallinarum* BSB 1 DSM 28103 ainsi que d'autres lactobacilles et levures formant ensemble un mélange de cultures de microorganismes ainsi que des produits de mouture de céréales.

3. Culture d'amorce selon la revendication 1 ou 2, comprenant comme mélange de cultures de microorganismes *Lactobacillus paracasei* BSB 2 DSM 28104 et *Lactobacillus gallinarum* BSB 1 DSM 28103, *Lactobacillus brevis, Lactobacillus delbrueckii, Lactobacillus plantarum, Saccharomyces cerevisiae,* ainsi que gruau de seigle, farine de seigle et eau.

4. Culture d'amorce selon au moins une des revendications 1 à 3, comprenant 0,005 à 0,2 % en poids, en particulier environ 0,02 % en poids du mélange de cultures de microorganismes, dont 0,005 à 0,2 % en poids, en particulier environ 0,018 % en poids de *Lactobacillus paracasei* BSB 2 DSM 28104 et *Lactobacillus gallinarum* BSB 1 DSM 28103, ainsi que 40 à 60 % en poids de gruau de seigle, en particulier 45 à 50 % en poids de gruau de seigle, 20 à 30 % en poids de farine de seigle, en particulier 25 % en poids de farine de seigle et 20 à 30 % en poids), en particulier 25 % en poids d'eau.

5. *Lactobacillus paracasei* BSB 2 DSM 28104.

6. *Lactobacillus gallinarum* BSB 1 DSM 28103.

7. Procédé de production d'une pré-pâte comme ingrédient de pâtes pour la préparation de produits de boulangerie et de pâtisserie à base de blé en utilisant un pourcentage de pain de blé comprenant les étapes suivantes :
a) dans une première étape, préparation d'un levain par mélange de 100 parties en poids de farine de blé avec 100 à 200 parties en poids d'eau et inoculation avec une culture d'amorce selon au moins une des revendications 1 à 4 épaissie avec des produits de mouture de céréales, dont la température se situe entre 20 à 35 °C et le premier mélange obtenu mûrit pendant une durée de 12 à 36 h,
b) dans une deuxième étape, production d'un mélange de farine de blé, d'un pourcentage de pain de blé et d'eau, comprenant une quantité de 75 à 135 parties en poids ; dont jusqu'à 15 à 75 parties en poids de pourcentage de pain de blé, le mélange étant mélangé à 150 parties en poids d'eau en malaxant en deux étapes, en particulier pour une durée de 10 à 120 min, dans lequel le produit possède une température dans une intervalle de température de 20 à 35 °C,
c) ajout d'une partie du levain obtenu dans la première étape conformément à l'étape a) à une plus grande partie du deuxième mélange pour amorcer la fermentation de la deuxième étape,
d) maturation du mélange combiné obtenu à l'étape c) pour une durée de 24 à 60 h, pour obtenir une prépâte comme ingrédient de pâtes pour la préparation de produits de boulangerie et de pâtisserie à base de blé.

8. Procédé selon la revendication 7, dans lequel la farine de blé est une farine de type 550 selon la norme DIN 10355, laquelle correspond à une teneur en cendres de 510 à 630 mg/100 g de farine, farine avec un degré de mouture de 640 à 900 mg/100 g.

9. Procédé selon au moins une des revendications 7 ou 8, dans lequel la deuxième étape combinée est laissé à maturation pour une durée de 20 à 48 h à 20 à 35 °C.

10. Procédé selon au moins une des revendications 7 à 9, dans lequel pour préparer la deuxième étape combinée 2,5 à 15,0 parties en poids de la première étape sont mélangées à 100 parties en poids du deuxième mélange.

11. Procédé selon l'une des revendications 7 à 10, dans lequel une valeur de pH de 3,5 s'établit au cours d'un processus reposant purement sur la fermentation et une stabilité au stockage de 144 heures au maximum sans refroidissement est obtenue.

12. Pré-pâte comme ingrédient de pâtes pour la préparation de produits de boulangerie et de pâtisserie à base de blé obtenue par un procédé selon la revendication 7.
